# EUROPEAN PATENT APPLICATION

(11) **EP 1 719 501 A1**
(43) Date of publication of application: **08.11.2006**
(21) Application number: 06009147.7
(22) Date of filing: 03.05.2006
(51) Int. Cl.: A61K 9/00, A61K 9/14

(54) **A powder composition comprising a mixture of a hydrophilic polymer matrix and a divalent inorganic metal**

(30) Priority: 05.05.2005 US 677989 P
(71) Applicant: National Starch and Chemical Investment Holding Corporation, New Castle, Delaware 19720 (US); UNIVERSITEIT GENT, 9000 Gent (BE)
(72) Inventor: Pringels, Eveline, 9070 Destelbergen-Heusden (BE); Remon, Jean Paul, 9090 Melle (BE); Vervaet, Chris, 8870 Izegem (BE); Foreman, Paul B., Sommerville NJ 08876 (US)
(74) Representative: Held, Stephan

(57) **Abstract**

The invention provides a safe drug delivery composition that enhances the absolute bioavailability of active agent (drug).

## Description

### FIELD OF THE INVENTION

The invention relates to compositions for the delivery of active agents, and to methods of preparing and using the composition, in particular for intranasal drug delivery.

### BACKGROUND OF THE INVENTION

Bioadhesion, also referred to as mucoadhesion, has been of interest for the development of controlled drug delivery systems to improve buccal, nasal and oral administration of drugs. The oral and nasal cavities, for example, form convenient and easily accessible sites for drug delivery.

Bioadhesive compositions are known to be useful for delivering drugs via the mucosa, especially macromolecular molecules that do not readily penetrate the skin or those which are problematic for peroral delivery (e.g., degrade in the gastro-intestinal tract, poor absorption through the intestinal mucosa).

The nasal mucosa comprises a highly vascularized epithelial layer. This site provides a convenient surface area suitable for self medication, thereby lowering the cost of therapy and increasing patient compliance. While the nasal route of administration provides advantages, there are also disadvantages. The active is subjected to mucociliary clearance and enzymes can diminish or remove the potency of the active agent.

There is thus a need in the art for compositions that enhance absorption of the active thereby increasing the bioavailability of the active. The current invention addresses this need.

### SUMMARY OF THE INVENTION

The invention encompasses compositions useful for delivering drugs via the mucosa, especially the nasal mucosa. Preferred compositions of the invention are powder formulations that comprise a mixture of a hydrophilic polymer matrix and a divalent metal inorganic compound. Divalent metal inorganic compounds preferred for use in the practice of the invention are compounds of calcium or magnesium, such as CaCO₃, Ca(OH)₂ and Mg(OH)₂. Preferably, the mixture will comprise from about 0.5% to abut 25 % by wt of said divalent inorganic metal compound and 99.5 % to about 75 % of by wt of said hydrophilic polymer matrix. In one embodiment the hydrophilic polymer matrix comprises from about 95 % to about 5 % by wt of a cross-linked poly(acrylic acid), from about 5 % to about 95 % by wt of a starch. The compositions of the invention may also, optionally, comprise an active ingredient.

Another embodiment of the invention relates to methods of preparing compositions for the delivery of active agents. In a preferred embodiment, a solution comprising at least one solvent and a polymer mixture is prepared, wherein the polymer mixture comprises at least one synthetic polycarboxylated polymer component and at least one polysaccharide component, the solution is dried to form a solid, preferably by spray-drying to form an intimate mixture of said components, and further mixing with said intimate mixture an amount of divalent inorganic metal compound effective to increase absorption of an active agent also incorporated therein or to be further incorporated therein prior to administration. In another embodiment, a solution comprising the solvent, polymer mixture, and divalent inorganic metal compound is prepared and the solution is spray-dried to form an intimate mixture. In yet another embodiment, a solution comprising the solvent, polymer mixture, divalent inorganic metal compound and the active ingredient(s) is prepared and the solution is spray-dried to form an intimate mixture.

Yet another embodiment of the invention is directed to an active component delivery vehicle comprising a mixture of a hydrophilic polymer matrix and a divalent metal inorganic compound and to methods of administering a therapeutic agent to an individual in need thereof. In one preferred embodiment, the delivery vehicle is a powder formulation that is applied to the nasal mucosal surface of the individual.

### BRIEF DESCRIPTION OF THE DRAWING FIGURES

Fig. 1 shows the insulin serum concentration-time profile after nasal administration of a powder formulation based on spray-dried Amioca® /Carbobol® 974P (25/75).
Fig. 2 is a graph showing the influence of CaCO₃ on the insulin bioavailability after nasal administration.
Fig. 3 is a graph showing the influence of the density of CaCO₃ (high versus low density) on the insulin bioavailability after nasal administration.
Fig. 4 is a graph showing the influence of the different Amioca® /Carbobol® 974P ratios (75/25 versus the 25/75 ratio of Figure 3) with and without the addition of CaCO₃ on the insulin bioavailability after nasal delivery.
Fig. 5 is a graph showing the influence of CaCO₃ on the calcitonin bioavailability after nasal administration.
Fig. 6 is a graph showing the influence of different Ca²⁺ compounds on the insulin bioavailability after nasal delivery.
Fig. 7 is a graph showing differences in insulin bioavailability after nasal administration when using a physical mixture of Amioca® /Carbobol® 974P instead of a spray-dried mixture of Amioca® /Carbobol® 974P with and without the addition of CaCO₃.
Fig. 8 is a graph showing the enzyme (LDH) activity in the nasal cavity of rabbits 1h before and 1h after nasal delivery during 5 consecutive days of a powder formulation based on a spray-dried mixture of Amioca® /Carbobol® 974P with and without the addition of CaCO₃.
Fig. 9 is a graph showing the enzyme (ALP) activity in the nasal cavity of rabbits 1h before and 1h after nasal delivery during 5 consecutive days of a powder formulation based on a spray-dried mixture of Amioca® /Carbobol® 974P with and without the addition of CaCO₃.
Fig. 10 is a graph showing the concentration of proteins released in the nasal cavity of rabbits 1h before and 1h after nasal delivery during 5 consecutive days of a powder formulation based on a spray-dried mixture of Amioca® /Carbobol® , 974P with and without the addition of CaCO₃over time using compositions with and without CaCO₃.
Fig. 11 is a graph showing the influence of Carbobol® 974P on absorption enhancement.

### DETAILED DESCRIPTION OF THE INVENTION

It has been surprisingly discovered that use of a hydrophilic delivery or carrier composition containing an effective amount of a divalent inorganic compound, such as CaCO₃, Ca(OH)₂, and Mg(OH)₂, increases the bioavailability of actives. Since it is widely believed that depletion of calcium or magnesium ions in the vicinity of the epithelium (with a carboxylated bioadhesive polymer, for example) is an enabling mechanism for enhanced paracellular transport of an active substance, it is surprising and unexpected that addition of such metal cations provides greatly enhanced bioavailability.

The invention provides safe carrier compositions for the delivery of active agents that increase the bioavailability of the active(s) incorporated therein. Increase in bioavailability of the active agent in amounts of up to 30% or more, generally at least an increase of about 18% or more, can be accomplished using the compositions of the invention.

The compositions are prepared from a mixture of at least one hydrophilic polymer and comprise at least one divalent metal inorganic cation. The invention also provides drug delivery systems, methods of preparing drug delivery systems, and methods of administering a therapeutic agent to an individual.

Solution, as used herein, is intended to mean partial solubilization, colloidal dispersion or total solubilization.

Solid, as used herein, is intended to mean a material having less than about 10 % by weight of solvent present, and includes powders.

Physical mixture is used herein to refer to mixtures comprising discrete particles of e.g., starch and poly(acrylic acid).

Intimate mixture is used herein to refer to mixtures wherein each particle comprises a mixture of, e.g., starch and poly(acrylic acid).

The compositions of the invention are used for the controlled release of active ingredient. The compositions may be used for both systemic and local administration of active ingredient.

The controlled release preparations of the invention find use in the administration of therapeutic agents to individuals in need of, or desirous of, the therapeutic agent. The term "individual" is used herein in its broadest sense and includes animals (both human and non-human, including companion animals such as dogs, cats and horses and livestock such as cattle and swine) and plants (both agricultural and horticultural applications being contemplated for use).

Controlled release, as used herein, is intended to mean a method and composition for making an active ingredient available to the biological system of a host. Controlled release includes the use of instantaneous release, delayed release, and sustained release. "Instantaneous release" refers to immediate release to the biosystem of the host. "Delayed release" means the active ingredient is not made available to the host until some time delay after administration. "Sustained Release" generally refers to release of active ingredient whereby the level of active ingredient available to the host is maintained at some level over a period of time. The method of affecting each type of release can be varied. For example, the active ingredient can be associated physically and/or chemically with a surfactant, a chelating agent, etc. Alternatively, the active ingredient can be masked by a coating, a laminate, etc. Regardless of the method of providing the desired release pattern, the present invention contemplates delivery of a controlled release system that utilizes one or more of the "release" methods and compositions. Moreover, the present invention can be an element of the release method and/or composition.

The composition of the present invention may take up and controllably release active components such as drugs. Active components may be added using any of the known methods described in the prior art, and such addition may be carried out during and/or after the production of the composition. Typical active components may include, but are not limited to, a therapeutic substance or a pharmaceutically active agent such as a drug, a non-therapeutic substance such as a cosmetic, a breath freshener and the like, a local or general anesthetic or pain killer, or an opiate, a vaccine, an antigen, a microorganism, a sterilizing substance, a contraceptive composition, a protein or peptide such as insulin or calcitonin, an insecticide, a herbicide, a hormone such as a growth hormone or a seed germination hormone, a steroid, a toxin, or a marker substance.

A non-limiting list of possible active components includes hydrochlorothiazide, acetazolamide, acetylsalicyclic acid, allopurinol, alprenolol, amiloride, antiarrhythmics, antibiotics, antidiabetics, antiepileptics, anticoagulants, antimycotics, atenolol, bendroflumethiazide, benzbromarone, benzthiazide, betamethasone, bronchodilators, buphenine, bupranolol, calcitonin, chemotherapeutics, chlordiazepoxide, chlorquine, chloro thiazide, chlorpromazine, chlortalidone, clenbuterol, clomipramine, clonidine, co-dergocrine, cortisone, dexamethasone, dextropropoxyphene, diazepam, diazoxide, diclofenac, diclofenamide, digitalisglycoside, dihydralazine, dihydroergotamine, diltiazem, ergotamine, ethacrynic acid, ethinylestradiol, ethoxzolamide, fenoterol, fludrocortisone, fluphenazine, fluorosemide, gallopamil, guanethidine, hormones, hydrochlorothiazide, hydrocortisone, hydroflumethiazide, insulin, immunosuppressives, ibuprofen, imipramine, indomethacine, coronartherapeutics, levodopa, lithium salt, magnesium salt, medroxyprogesteron acetate, manadione, methaqualone, 8-methoxypsoralen, methylclothiazide, methyldopa, methylprednisolone, methyltestosterone, methylthiouracil, methylxanthine, metipranolol, miconazole nitrate, molsidomine, morphine, naproxen, nicergline, nifedipine, norfenefrine, oxyphenbutazone, papaverine, parmathasone, pentobarbital, perphenazine, phenobarbital, phenylbutazone, phytomenadione, pirenzepine, polythiazide, prazosine, prednisolone, prednisone, probenecid, propranolol, propylthiouracil, rescinnamine, reserpine, secbutabarbital, secobarbital, spironolactone, sulfasalazine, sulfonamide, testosterone, theophylline, thioridazine, triamcinolon, triamteren, trichloromethiazide, trifluoperazine, trifluopromazine, tuberculostatic, verapamil, virustatics, zytostatics, bromocriptine, bromopride, carbidopa, carbocromen, quinine, chlorprothixene, cimetidine, clofibrat, cyclizine, desipramine, disulfiram, domperidone, doxepine, fenbufen, flufenamine acid, flunarizine, gemfibrocil, haloperidol, ketoprofen, labetalol, lorazepam, mefenamine acid, melperone, metoclopramide, nortriptyline, noscapine, oxprenolol, oxymetholone, pentazocine, pethidine, stanozolol, sulindac, sulpiride, tiotixen.

The compositions of the invention may be prepared by forming a solution comprising at least one solvent and a polymer mixture (i.e., the hydrophilic polymer matrix components). In a preferred embodiment, the polymer mixture comprises at least one synthetic polycarboxylated polymer component and at least one polysaccharide component, the solution is then dried to form a solid, preferably by spray-drying to form an intimate mixture of said components, and further physically mixing with said intimate mixture an amount of divalent inorganic metal compound effective to increase absorption of an active agent also incorporated therein or to be further incorporated therein prior to administration. After physical mixing the hydrophilic polymer matrix with the divalent inorganic metal compound a dispersion is made which is (or can be) neutralized followed by adding the active agent. The dispersion can then be freeze-dried to remove the water content.

In another embodiment, a solution comprising the solvent, polymer mixture, and divalent inorganic metal compound is prepared and the solution is spray-dried to form an intimate mixture.

In yet another embodiment, a solution comprising the solvent, polymer mixture, divalent inorganic metal compound and the active ingredient(s) is prepared and the solution is spray-dried to form an intimate mixture.

The compositions of the invention are particularly well suited for delivery of macromolecules via the nasal mucosa.

The compositions of the invention may optionally contain one or more absorption enhancers. The absorption enhancers are incorporated in the composition by drying a solution of at least one solvent, at least one absorption enhancer, a synthetic polycarboxylated polymer and a polysaccharide to form a solid composition. Useful absorption enhancers, also referred to as permeation enhancers, include, but are not limited to, synthetic surfactants (e.g. sodium lauryl sulphate), non-ionic surfactants (e.g. laureth, polysorbate), steroidal surfactants (e.g. sodium cholate), bile salts (e.g. sodium glycocholate, sodium deoxycholate, sodium taurocholate), chelators (e.g. EDTA, disodium EDTA), fatty acids and derivatives (e.g. sodium myristate), others as sugar esters (e.g. sucrose palmitate), phosphatidylcholine, aminocaproic acid, lauramidopropylbetaine, etc. Amounts of absorption enhancers incorporated into the compositions of the invention will typically range from about 0.001 % by weight to about 10 % by weight.

While the invention will be described in detail with respect to carrier compositions comprising starch and polyacrylic acid, it is to be understood that the invention is not to be so limited. Any hydrophilic polymer matrix may be used in the practice of the invention. Hydrophilic polymers include water soluble and water dispersible polymers such as but not limited to hydroxypropylmethyl cellulose, hydroxyethyl cellulose, hydroxypropyl cellulose, polyvinyl pyrrolidone, carboxymethyl cellulose, polyvinyl alcohol, sodium alginate, polyethylene glycol, xanthan gum, gum tragacanth, guar gum, acacia gum, arabic gum, polyacrylic acid, methylmethacrylate copolymer carboxyvinyl polymer and copolymers and mixtures thereof.

Preparation of the compositions of the invention may be accomplished by charging solutions in at least one solvent, preferably water, of a polysaccharide and a synthetic polycarboxylated polymer into a mixing vessel and stirring until uniformly mixed. It may be necessary, as for example in the case of AMIOCA, to heat the component polymer and its solvent(s) in order to obtain solution. A partial solution, obtained for example via a batch cooker, may be used provided there is sufficient dissolution for intimate mixing with the polycarboxylated polymer. In other cases it may be possible to dissolve and mix both polymers in a single step. The polycarboxylated polymer may be neutralized with a base, such as NaOH, to effect changes in viscosity, as would be apparent to one skilled in the art. The concentrations of the component polymer solutions are determined only by consideration of degree of solubility and a convenient viscosity for mixing and subsequent processing, as will be obvious to one skilled in the art.

The ratio of polymers in the solution mixture lies within the range of about 5 parts (on a dry weight basis) polysaccharide plus 95 parts polycarboxylated polymer to about 95 parts polysaccharide plus 5 parts polycarboxylated polymer. In one preferred embodiment the ratio of polymers in the solution is about 25 parts polysaccharide to about 75 parts polycarboxylated polymer.

The mixture is then dried by conventional means, including, but not limited to, spray drying, freeze drying, air drying, drum drying and extrusion, to provide a solid (e.g., a powder). The solid produced during the drying stage will preferably have a moisture content of less than about 10 % by weight. A particularly preferred method is spray drying, which forms an intimate mixture of the polymers.

The conditions used to prepare the compositions of the invention are sufficiently mild and/or the processing sufficiently rapid that unwanted chemical reactions, that may lead to deleterious by-products, are avoided. Thus, no purification step is needed to remove such components.

The carrier compositions, e.g., prior to incorporation of an active ingredient, may be neutralized by known means, if desired.

The synthetic polycarboxylated polymers of this invention may be modified or unmodified and have a weight average molecular weight of at least 10,000 Daltons, more typically at least about 100,000 Daltons, even more typically above about 1,000,000 Daltons. Modifications may include, but are not limited to cross-linking, neutralization, hydrolysis and partial esterification.

Exemplary synthetic polycarboxylated polymers which may be used in the present invention include without limitation poly(acrylic acid), cross-linked poly(acrylic acid), poly(acrylic acid) modified by long chain alkyl acrylates, cross-linked poly(acrylic acid) modified by long chain alkyl acrylates. Typical synthetic polycarboxylated polymers of this invention include acrylic acid polymers crosslinked with allyl sucrose, allyl ethers of sucrose, allylpentaerythritol, pentaerythritol or divinyl glycol. Such polymers are available from Noveon Company, Cleveland, Ohio, USA under the trade names CARBOPOL® , NOVEON® and PEMULEN® . Particularly suitable are the pharmaceutical grades CARBOPOL® 971P, CARBOPOL® 934P and CARBOPOL® 974P. These examples are not limiting and the polysaccharides according to the present invention may be used in combination with virtually any synthetic polycarboxylated polymer.

The polysaccharides of the present invention are derived from natural products, including plant, animal and microbial sources. Examples of polysaccharides include starch, cellulose and gums such as galactomannans. Polysaccharide starches include maize or corn, waxy maize, potato, cassava, tapioca and wheat starch. Other starches include varieties of rice, waxy rice, pea, sago, oat, barley, rye, amaranth, sweet potato, and hybrid starches available from conventional plant breeding, e.g., hybrid high amylose starches having amylose content of 40 % or more, such as high amylose corn starch. Also useful are genetically engineered starches such as high amylose potato and waxy potato starches. The polysaccharides may be modified or derivatized, such as by etherification, esterification, acid hydrolysis, dextrinization, crosslinking, pregelatinization or enzyme treatment (e.g., with *alpha-*amylase, beta-amylase, pullulanase, isoamylase, or glucoamylase). Particularly preferred are waxy starches. As used herein, the term "waxy" is intended to include a starch containing at least about 95 % by weight amylopectin.

Preferred polysaccharides will have a weight average molecular weight of at least 10,000 Daltons, more preferably at least about 100,000 Daltons, even more preferably above about 500,000 Daltons, and most preferably greater than about 1,000,000 Daltons. While molecular weights of waxy starches are difficult to determine, waxy starches that can be used in the practice of the invention may have weight average molecular weights of 10,000,000 Daltons or more.

The invention will be described further in the following examples, which are included for purposes of illustration and are not intended, in any way, to be limiting of the scope of the invention.

### EXAMPLES

### Example 1

### Preparation of hydrophilic polymer matrices

A mixture of 10% by weight of AMIOCA waxy corn starch (obtained from National Starch & Chemical Company, Bridgewater, New Jersey) and 90% water was prepared as a slurry. The mixture was heated by injecting steam at a pressure of 2.75 bar in a continuous jet cooker, maintaining the temperature at 150°C by adjustment with jacketed cooling water. The final starch solids content, determined by heating a small sample for 2 hours at 135°C, was 7.74%.

A 1 % aqueous solution of CARBOPOL® 974P (obtained from B.F. Goodrich Company) was prepared by slowly adding the CARBOPOL to deionized water while continuously stirring until completely dispersed.

The starch and CARBOPOL solutions were uniformly mixed in such proportions as to obtain the desired ratio of starch to CARBOPOL. For example, mixing 1085 g AMIOCA solution with 5600 g CARBOPOL solution yielded a ratio of 60% AMIOCA to 40% CARBOPOL, calculated on solids basis. The solution mixture was heated in a water bath to 40°C and spray dried using a centrifugal wheel atomizer. The inlet temperature during drying was 205°C and the outlet temperature 110°C. The resulting product was a fine, low density, white powder comprising an intimate mixture of AMIOCA and CARBOPOL. In this example the sample is designated SD 60/40 to indicate preparation by spray drying in a ratio of 60% AMIOCA to 40% CARBOPOL. Other ratios, as used in the following examples, are similarly designated.

In the Example 9, in which the starch and CARBOPOL were blended as solids to form a physical mixture are prefixed PM. The physical mixtures were prepared by dry blending AMIOCA with CARBOPOL.

### EXAMPLE 2

### Nasal delivery of peptides and proteins

After partial neutralization (partial neutralization of the comixture prevents denaturing of insulin and also permits use of high levels of Carbopol without inducing irritation)of a dispersion of SD 25/75 with NaOH in distilled water to a pH of 7.4, insulin was added in a ratio of 1IU insulin per mg powder. This was lyophilized and the powder was then passed through a sieve of 63µm mesh size. 10 mg of the powder was given to rabbits per nostril (n = 8).

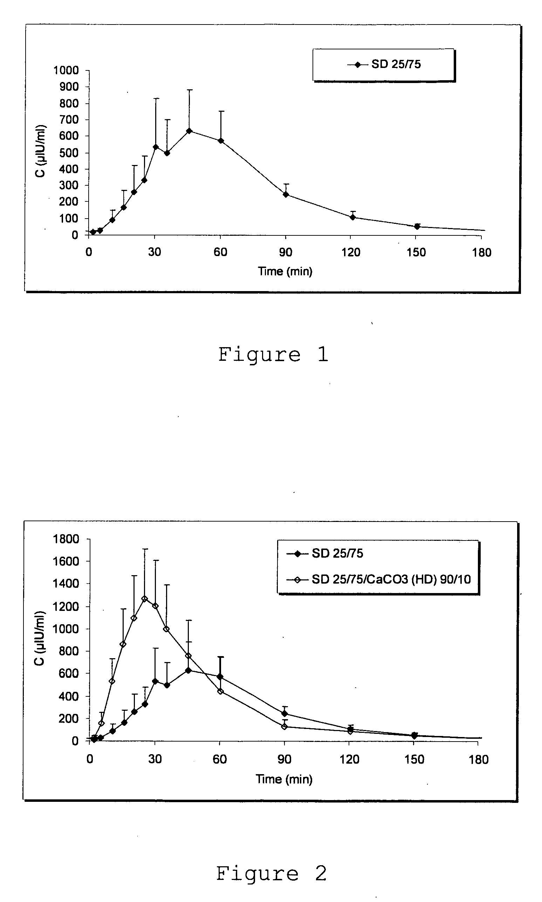

As can be seen in Fig. 1, the insulin serum concentration (Cₘₐₓ) was 681.0 ± 247 µIU/ml and tₘₐₓ was 51 ± 7 min. The absolute insulin bioavailability was calculated to be 19.2 ± 5.3 %, which clearly demonstrates that peptides and proteins can be delivered via the nasal mucosa from the hydrophilic matrix compositions of the invention.

### EXAMPLE 3

### Effects of incorporation of high density calcium carbonate into the hydrophilic matrix on insulin bioavailability

A powder formulation was prepared based on a physical mixture of 90 % SD 25/75 and 10% CaCO₃ HD. Results are shown in Table 1 and in Fig. 2.

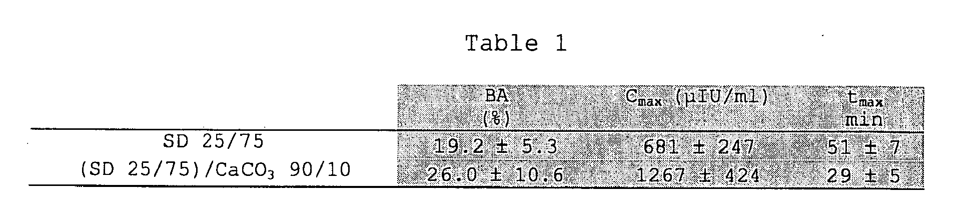

Fig. 2 shows the insulin serum concentration-time profiles after nasal delivery to rabbits of powder formulations based on a mixture of spray-dried Amioca® starch/Carbopol® 974P 25/75 and on a mixture of (spray-dried Amioca starch/Carbopol 974P 25/75)/CaCO_{3HD} 90/10. As can be seen in Fig. 2, nasal delivery of this formulation resulted in a Cₘₐₓ of 1267±424 µIU/ml and tₘₐₓ of 28.9±5.1 min.

This example shows that the calculated bioavailability of insulin increased to 26.0± 10.6% when CaCO_{3HD} was used in the formulation. Bioavailability increased, Cₘₐₓ increased and tₘₐₓ decreased when CaCO_{3HD} was incorporated into the composition.

### Example 4

### Influence of density of calcium carbonate

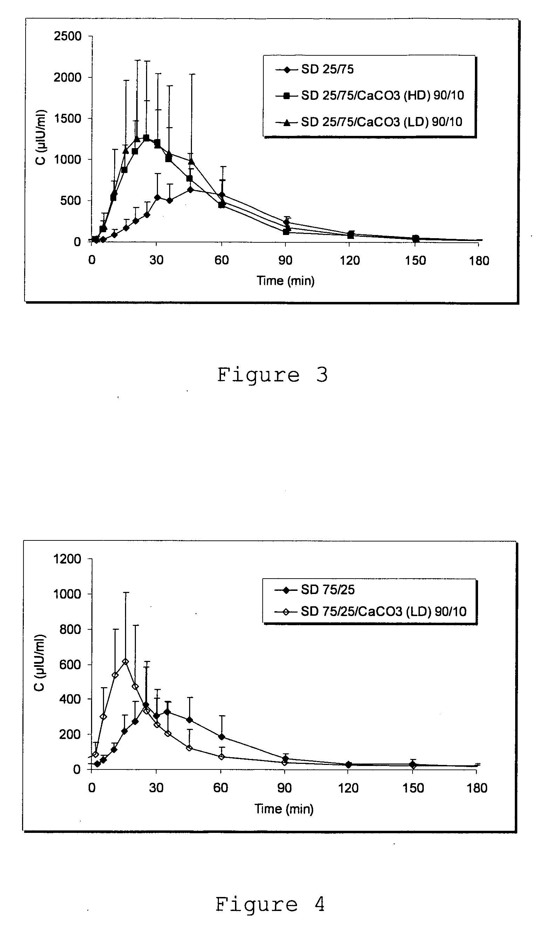

Example 3 was repeated using low density calcium carbonate (CaCO_{3LD}). As can be seen in Fig. 3, use of CaCO_{3LD} had a similar bioavailability, Cₘₐₓ and tₘₐₓ.

### Example 5

### Influence of hydrophilic matrix composition

Example 4 was repeated except that instead of a SD (25/75) /CaCO_{3LD} 90/10 mixture, a (75/25) /CaCO_{3LD} 90/10 mixture was prepared and tested. Results are shown in Table 2 and in Fig. 4.

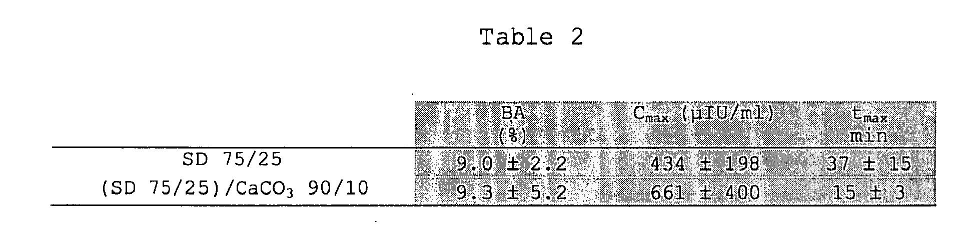

Results show that while the bioavailability of insulin is similar, there is an increase in Cₘₐₓ and a decrease in tₘₐₓ.

### Example 6

### Effects of incorporation of low density calcium carbonate into the hydrophilic matrix on calcitonin bioavailability

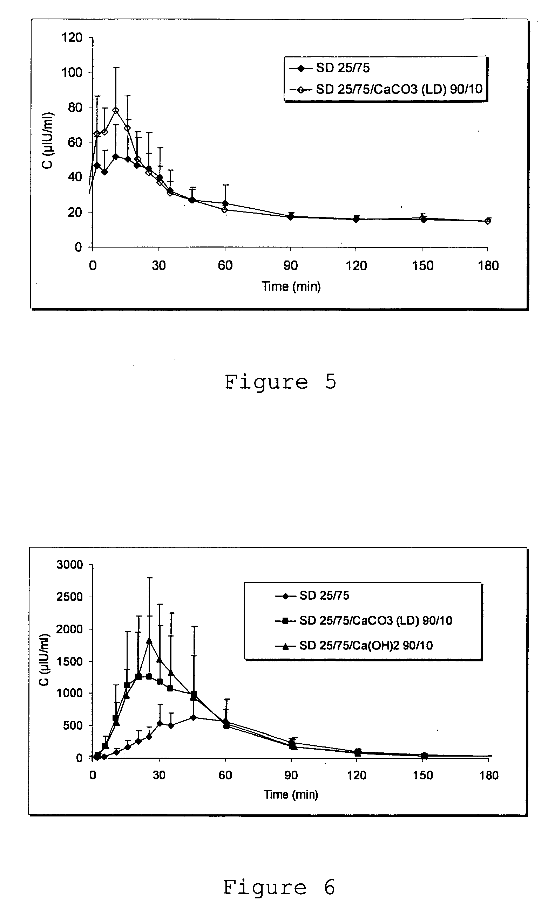

Example 4 was repeated except that insulin was replaced with calcitonin. From Fig. 5 it can be seen that use of CaCO_{3LD} in the formulation resulted in a slightly higher bioavailability, an increased Cₘₐₓ and a similar tₘₐₓ.

### Example 7

### Influence of different Ca2+ compounds on absorption enhancement

Example 4 was again repeated except that CaCO_{3LD} was replaced with the more soluble Ca(OH)₂. As can be seen in Fig. 6, addition of 10 % Ca(OH)₂ to 90 % SD 25/75 has a similar release profile as 90 (SD 25/75)/10 CaCO₃.

### Example 8

### Influence of ratio of Ca2+ ions used in the hydrophilic matrix composition

Example 7 was repeated using various amounts of Ca(OH)₂ ((SD25/75)/Ca(OH)₂ ratios of 90/1, 90/10, 90/20 and 90/30). Results are show in Table 3.

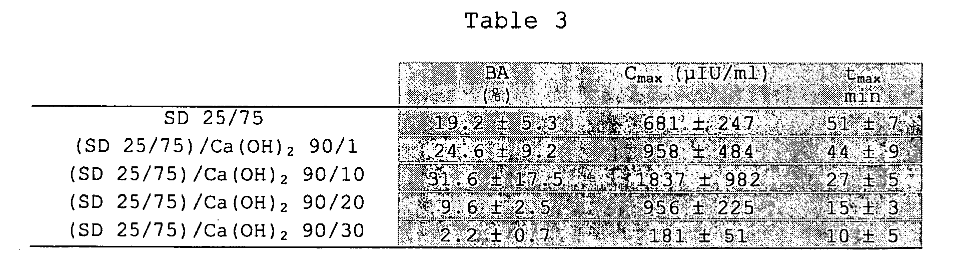

From these results it is seen that optimum bioavailability and Cₘₐₓ is seen for the 90/10 formulation. Tₘₐₓ decreases as the concentration of Ca(OH)₂ increases.

### Example 9

### Physical mixtures versus spray dried mixtures

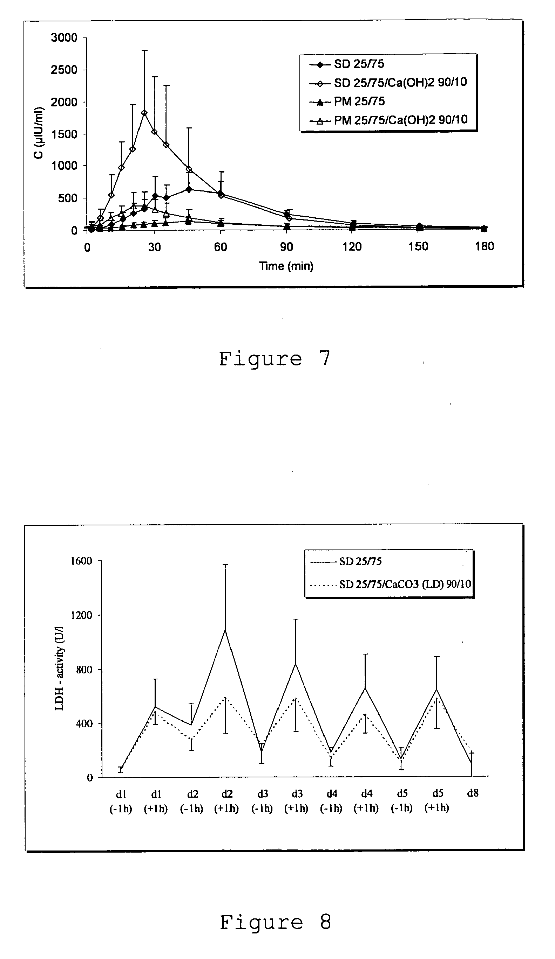

Example 7 was repeated except that the SD 25/75 was replaced with a physical mixture of Amioca and Carbopol. The comparison of compositions comprising a physical mixture or spray-dried mixtures alone, and (90 PM 25/75)/10 Ca(OH)₂ versus (90 SD 25/75)/10 Ca(OH)₂ is shown in Fig. 7. From Fig. 7 it is seen that compositions made with a physical mixture show a decrease in insulin uptake but when Ca(OH)₂ is added a similarly enhanced release profile is observed.

### Example 10

### Influence of other divalent metal cations

Example 7 was repeated using Mg(OH)₂ instead of Ca(OH)₂. The results, set forth in Table 4, show that addition of Mg(OH)₂ to SD 25/75 has a similar bioavailability, Cₘₐₓ and tₘₐₓ as (SD 25/75) /Ca(OH)₂.

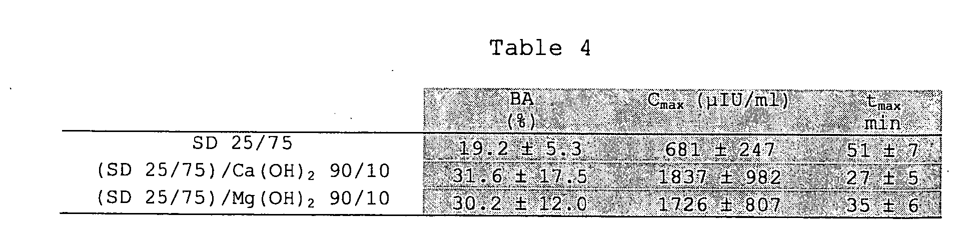

### Example 11

### Irritation potential of calcium carbonate

Analysis of protein and enzyme release from the mucosa after treatment, provides a measure of irritation potential. Release of membrane-bound alkaline phosphatase (ALP) and also the appearance of the cytosolic enzyme lactate dehydrogenase (LDH) result from damaged cells.

In this example SD 25/75 was compared to (SD 25/75)/CaCO_{3LD'} For five consecutive days, 10,0 mg of powder was sprayed into the nostrils of rabbits. Nostrils were washed with phosphate buffered saline 1 hour before and 1 hour after administration and the amount of LDH and ALP tested to determine membrane damage.

From Fig. 8 it is seen that for both formulations the level of LDH activity increases 1 hour after administration. Decreased LDH activity is seen 23 hours after administration. It is also seen that LDH activity for SD 25/75 is higher than LDH activity using (SD 25/75)/CaCO₃. Notably, 3 days after the last administration, LDH release is comparable with basal LDH release indicating that the membrane damage is reversible.

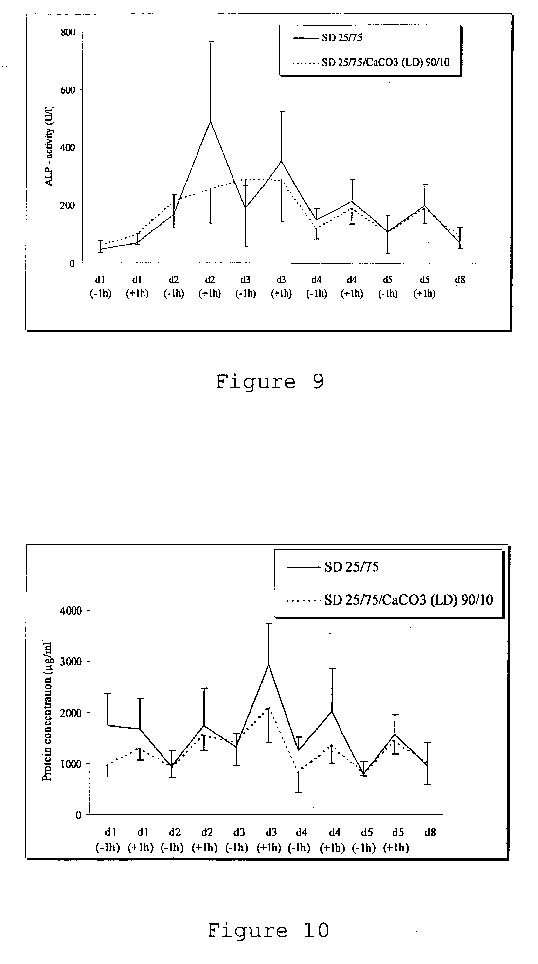

From Fig. 9 it is seen that with both formulations the level of ALP activity increases 1 hour after administration. Decreased ALP activity is seen 23 hours after administration. It is also seen that ALP activity for SD 25/75 is comparable to ALP activity using (SD 25/75)/CaCO₃. Three days after the last administration, ALP release is comparable with basal LDH release indicating that the membrane damage is reversible.

From Fig. 10 it is seen that protein release follows a similar trend to enzyme release.

### Example 12

### Influence of Carbopol® 974 on absorption enhancement

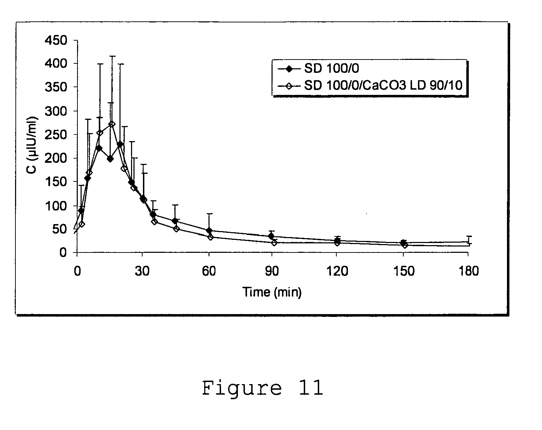

Example 4 was repeated except that the pure Amioca starch was used in place of the Amioca/Carbopol spray dried mixture. As can be seen in Fig. 11, use of CaCO_{3LD} in the formulation resulted in similar bioavailability, Cₘₐₓ and tₘₐₓ.

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

## Claims

1. A powder composition comprising a mixture of a hydrophilic polymer matrix and a divalent inorganic metal compound, wherein the hydrophilic matrix comprises at least one synthetic polycarboxylated polymer.

2. The composition of claim 1 wherein the metal is calcium or magnesium.

3. The composition of claim 2 wherein the mixture comprises from about 0.5% to about 25 % by wt of said divalent inorganic metal compound.

4. The composition of claim 3 wherein said divalent inorganic metal compound is selected from the group consisting of CaCO₃, Ca(OH)₂, Mg(OH)₂ and mixtures thereof.

5. The composition of claim 4 wherein the hydrophilic matrix comprises from about 5 % by weight to about 95 % by weight of said at least one synthetic polycarboxylated polymer and from about 5 % by weight to about 95 % by weight of at least one polysaccharide.

6. The composition of claim 5 comprising from about 5 % by weight to about 35 % by weight of said at least one polysaccharide and from about 50 % by weight to about 95 % by weight of said at least one synthetic polycarboxylated polymer.

7. The composition of claim 6 wherein the polysaccharide is a starch and the polycarboxylated polymer is a cross-linked poly(acrylic acid).

8. . The composition of claim 7 wherein the starch is a waxy starch.

9. The composition of claim 3 further comprising an active agent.

10. The composition of claim 9 wherein the active agent is a polypeptide.

11. A method of producing a powder composition containing at least one synthetic polycarboxylated polymer component, at least one polysaccharide component, and at least one divalent inorganic metal compound, said method comprising preparing a solution comprising at least one solvent and a polymer mixture wherein the polymer mixture comprises at least one synthetic polycarboxylated polymer component and at least one polysaccharide component, and then drying the solution to form a solid comprising an intimate mixture of said components, wherein the said at least one divalent inorganic metal compound is added to the solution prior to drying and is part of said intimate mixture or is physically mixed with said intimate mixture following the drying thereof.

12. The method of claim 11 further comprising adding an active ingredient, wherein the active ingredient is added to the solution prior to drying and becomes part of said intimate mixture or is physically mixing with said intimate mixture following the drying thereof.

13. The method of claim 11 wherein said polymer mixture comprises, on a solids basis, from about 5 % by weight to about 35 % by weight of at least one polysaccharide and from about 50 % by weight to about 95 % by weight of at least one synthetic polycarboxylated polymer.

14. The method of claim 11 wherein the solution is spray-dried.

15. The method of claim 14 wherein the solvent is water, the polysaccharide is a starch and the polycarboxylated polymer is cross-linked poly(acrylic acid).

16. A composition prepared by the method of claim 11.

17. An active component delivery vehicle comprising the composition of claim 1 and an active ingredient.

18. The delivery vehicle of claim 17 wherein the composition comprises from about 75 % by weight to about 99.5 % by weight of said polymer matrix and from about 0.5% to about 25 % by wt of said divalent inorganic metal compound, said polymer matrix comprising from about 75 % by weight to about 95 % by weight of a cross-linked poly(acrylic acid) and from about 5 % by weight to about 25 % by weight of a starch.

19. A method of administering a therapeutic agent to an individual in need thereof comprising administering a drug delivery system comprising the composition of claim 1 and an active agent.

20. The method of claim 19 wherein the composition comprises from about 75 % by weight to about 99.5 % by weight of said polymer matrix and from about 0.5% to about 25 % by wt of said divalent inorganic metal compound, said polymer matrix comprising from about 75 % by weight to about 95 % by weight of a cross-linked poly(acrylic acid) and from about 5 % by weight to about 25 % by weight of a starch.

21. The method of claim 20 wherein the composition is applied to a mucosal surface of the individual.

22. The method of claim 21 wherein the composition is applied to the nasal mucosa.
